# EUROPÄISCHE PATENTANMELDUNG

(11) **EP 1 640 037 A1**
(43) Veröffentlichungstag der Anmeldung: **29.03.2006**
(21) Anmeldenummer: 04022962.7
(22) Anmeldetag: 27.09.2004
(51) Int. Cl.: A61N 5/06

(54) **Lampe zur Behandlung der Haut**

(71) Anmelder: WaveLight Laser Technologie AG, 91058 Erlangen (DE)
(72) Erfinder: Winkler, Martin, 09337 Hohenstein-Ernsttal (DE); Busch, Christian, 90765 Fürth (DE); Vogler, Klaus Dr., 90542 Eckental (DE)
(74) Vertreter: von Hellfeld, Axel

(57) **Zusammenfassung**

Eine Lampe zur therapeutischen oder/und kosmetischen Behandlung der Haut umfasst eine Breitband-Strahlungsquelle (20), eine Reflektionsfilteranordnung (22), welche einen Teil der von der Strahlungsquelle (20) erzeugten Strahlung entlang eines Nutzstrahlengangs (24) reflektiert, sowie eine Strahlfalle, welche die durch die Reflektionsfilteranordnung (22) durchgehende Strahlung absorbiert. Die Strahlfalle umfasst bei einer bevorzugten Ausführungsform profilierte Absorptionsflächen (40, 42), deren Profilierung von Dreiecksrillen gebildet ist. Die Reflektionsfilteranordnung (22) ist bevorzugt von zwei entlang des Nutzstrahlengangs (24) hintereinander angeordneten Reflektionsspiegeln (30, 32) gebildet, welche im Nutzband reflektierend sind, jedoch für unerwünschte Strahlungsanteile transmissiv sind. Die Absorptionsflächen (40, 42) sind bei der bevorzugten Ausführungsform unmittelbar unterhalb der Reflektionsspiegel (30, 32) angeordnet.

## Beschreibung

Die Erfindung betrifft eine Lampe zur Behandlung der Haut, beispielsweise zur Behandlung von Psoriasis, Vitiligo und anderen entzündlichen Hautkrankheiten sowie für kosmetische Anwendungen.

Für medizinische und kosmetische Anwendungen in der Dermatologie sind Lasergeräte weit verbreitet. Klinische Untersuchungen haben gezeigt, dass für verschiedene Indikationen verschiedene Wellenlängen besonders günstig sind (z.B. 308 nm bei Psoriasis, 750 nm für Epilation, 3 µm zur Hautverjüngung). Lasergeräte sind technologisch bedingt relativ teuer und erfordern eine gehobene Fachkenntnis bei der Benutzung, um eine Gefährdung der Patienten auszuschließen.

Daher ist versucht worden, die spektral selektive Wirkung von Lasergeräten, die unter anderem auf deren Monochromasie beruht, mit einfacheren und preiswerteren Lichtquellen nachzubilden. Beispielsweise sind sogenannte IPL-Systeme *(*IPL*=Intensive Pulsed Light)* zur Anwendung gekommen, die herkömmliche Blitzlampen verwenden.

Blitzlampensysteme besitzen eine vergleichsweise brandbandige Lichtemission, die sich vom ultravioletten Bereich über den sichtbaren Bereich bis hin in den infraroten Bereich erstrecken kann. Um unerwünschte Wirkungen der Hautbestrahlung zu vermeiden, ist es erforderlich, aus dem breitbandigen Emissionsspektrum solcher Blitzlampensysteme einen gewünschten nutzbaren Wellenlängenbereich zu selektieren. Die bei bekannten IPL-Systemen eingesetzten Maßnahmen zur spektralen Selektion eines geeigneten Wellenlängenbereichs sind größtenteils unzureichend und ineffizient. Im Rahmen dieser Maßnahmen werden üblicherweise sogenannte "Cut-off"-Filter verwendet, die Wellenlängenbereiche unterhalb einer sogenannten "Cut-off"-Wellenlänge (Grenzwellenlänge) mehr oder weniger gut unterdrücken. Bei diesen "Cut-off"-Filtern handelt es sich um Farbglasfilter, die kurzwelligere Spektralanteile unterhalb der Grenzfrequenz unterdrücken, längerwellige Spektralanteile bis zur langwelligen Emissionsgrenze der verwendeten Strahlungsquelle jedoch im wesentlichen ungeschwächt durchlassen. Eine Selektion schmalbandiger Wellenlängenbereiche ist mit solchen Farbglasfiltern nicht möglich.

Es sind außerdem schmalbandige Bandpassfilter bekannt, mit denen sich ein vergleichsweise eng begrenzter Wellenlängenbereich von beispielsweise 15 oder 20 nm selektieren lässt. Solche Bandpassfilter (Interferenzfilter) haben sich jedoch als ungeeignet erwiesen, um bei Blitzlampensystemen, die für die Behandlung der Haut ausgelegt und bestimmt sind, eine Spektralselektion herbeizuführen. Die hohe Absorption herkömmlicher Bandpassfilter außerhalb des Transmissionsbereichs führt nämlich zu einer vergleichsweise raschen Erwärmung und letztendlich zur thermischen Zerstörung der Filter. Dies ist besonders ungünstig vor dem Hintergrund des hohen Preises solcher Filter. Zudem besitzen Interferenzfilter in ihrem Transmissionsbereich oftmals lediglich einen Transmissionsgrad von 20 - 40%.

Aufgabe der Erfindung ist es daher, eine konstruktiv einfach und preisgünstig herstellbare Lampe zur Hautbehandlung zu schaffen, die durch eine wirksame spektrale Selektion aus einer breitbandigen Strahlungsquelle eine wenigstens näherungsweise monochromatische (quasi-monochromatische) Lichtquelle macht und gleichzeitig vor thermischen Schäden infolge der Absorption unerwünschter Wellenlängen gut geschützt ist.

Zur Lösung dieser Aufgabe ist erfindungsgemäß eine Lampe zur Behandlung der Haut vorgesehen, wobei die Lampe eine Breitband-Strahlungsquelle, eine Reflexionsfilteranordnung, welche einen Teil der von der Strahlungsquelle erzeugten Strahlung entlang eines Nutzstrahlengangs reflektiert, sowie eine Strahlfalle umfasst, welche die durch die Reflexionsfilteranordnung durchgehende Strahlung absorbiert. Bei der erfindungsgemäßen Lösung gelingt es, durch Verwendung eines oder mehrerer Reflexionsfilter die breitbandige Emissionscharakteristik einer breitbandigen Strahlungsquelle, bei der es sich beispielsweise um eine Xenon-Blitzleuchte handeln kann, spektral selektiv auf ein schmalbandiges, quasi-monochromatisches Emissionsgebiet einzuengen. Schmalbandig bedeutet beispielsweise ein Wellenlängenband mit einer Breite von 5 - 40nm, wobei es sich versteht, dass diese Zahlenwerte in keinerlei Hinsicht als beschränkend anzusehen sind. Reflexionsfilter sind mit Reflexionsgraden von bis zu 100% in einem gewünschten Reflexionsband und Transmissionsgraden von zum Teil über 90% für den überwiegenden Rest der Strahlung handelsüblich erhältlich. Angesichts dieser Charakteristiken erlauben Reflexionsfilter einerseits eine sehr effiziente Fiiterung eines interessierenden Wellenlängenbereichs, andererseits ist eine thermische Beschädigung der Filter nahezu ausgeschlossen. Die Bandbreite der am Ausgang der Reflexionsfilteranordnung bereitstehenden Nutzstrahlung lässt sich relativ einfach durch das Design der verwendeten Reflexionsfilter einstellen.

Der unerwünschte Anteil der von der Strahlungsquelle emittierten Strahlung wird bei der erfindungsgemäßen Lösung durch die Reflexionsfilteranordnung von der Nutzstrahlung separiert und in der Strahlfalle abgefangen. Durch geeignete Ausgestaltung der Strahlfalle kann eine hochwirksame Absorption des nicht genutzten Strahlungsanteils erreicht werden, wodurch die erfindungsgemäße Lampe insgesamt sehr robust und unempfindlich gegenüber thermischen Belastungen ist.

Die erfindungsgemäße Lösung erlaubt es, eine sehr hohe spektrale Reinheit zu erreichen, d.h. Spektralanteile unerwünschter Wellenlängenbereiche können sehr effizient unterdrückt werden. Auch spektrale Transmissions-"Resonanzen", wie sie bei herkömmlichen Interferenzfiltern aufgrund periodisch wiederkehrender Transmissionsbereiche auftreten, werden nicht beobachtet.

Durch geeignete Wahl der Filtercharakteristik der Reflexionsfilteranordnung ermöglicht es die erfindungsgemäße Lösung, im wesentlichen beliebige spektrale Bereiche eines breitbandigen Emitters frei auszuwählen und in einem sehr breiten Spektralbereich vom Ultravioletten bis hin zum Niederinfraroten (NIR) schmalbandige, quasimonochromatisch strahlende Lampen zu realisieren. Im Unterschied zu teuren Interferenzfiltern schwächen Reflexionsfilter den ausgewählten Nutzspektralbereich kaum. Durch Verwendung von Standardkomponenten ist die erfindungsgemäße Lampe preisgünstig realisierbar.

Bei einer bevorzugten Ausführungsform weist die Strahlfalle mindestens eine profilierte Absorptionsfläche auf. Die Profilierung der Absorptionsfläche wird so gewählt, dass die auftreffende unerwünschte Strahlung nicht in den Nutzstrahlengang zurückkehrt. Vorzugsweise wird eine derartige Profilierung der Absorptionsfläche gewählt, dass sich die abzufangende Strahlung sozusagen in der Profilierung verliert. Hierzu kann beispielsweise in die Absorptionsfläche eine Vielzahl von parallel nebeneinander verlaufenden Rillen eingeformt sein. Als besonders vorteilhaft hat sich im Querschnitt eine Dreiecksform der Rillen gezeigt, wobei ein Öffnungswinkel der Dreiecksrillen zwischen 45 und 75°, vorzugsweise etwa 60°, besonders gute Ergebnisse ergeben hat.

Die mindestens eine Absorptionsfläche ist bevorzugt an der Innenwand eines Lampengehäuses, insbesondere aus Aluminium, ausgebildet, welches von einer Kühlkanalanordnung durchsetzt ist und Kühlmittelschnittstellenanschlüsse für den Anschluss einer Kühlmittelversorgung aufweist. Indem ein Kühlmittel durch die Kühlmittelkanalanordnung des Lampengehäuses geleitet wird, kann die Temperatur des Lampengehäuses auf einem akzeptablem Niveau von beispielsweise etwa 40°C gehalten werden.

Grundsätzlich ist es vorstellbar, die Reflexionsfilteranordnung von einem einzigen Reflexionsfilter zu bilden. Vorzugsweise umfasst die Reflexionsfilteranordnung jedoch zwei oder mehr Reflexionsfilter. Mehrere Reflexionsfilter erlauben es, einen besonders scharf begrenzten Wellenlängenbereich aus dem Spektrum der Strahlungsquelle zu selektieren. Auch kann durch Verwendung mehrerer Reflexionsfilter der Aufwand für die einzelnen Filter gering gehalten werden. Es müssen nicht aufwendige Reflexionsspiegel mit extrem hoher Schichtzahl hergestellt werden, die zumeist weniger strahlungsresistent als Filter mit einer geringeren Schichtzahl sind. Durch eine leichte Verstimmung der Mittenfrequenz zweier Reflexionsfilter lässt sich zudem eine besonders geringe Bandbreite der von der Lampe abgegebenen Nutzstrahlung einstellen.

Konstruktiv einfach kann an zwei schräg zueinander verlaufenden Innenwandflächen eines Lampengehäuses der Lampe je ein Reflexionsfilter angeordnet sein, wobei die Reflexionsfilter beispielsweise in Ausnehmungen in den Innenwandflächen gehalten sein können. Die unter den Reflexionsfiltern befindlichen Bereiche der Innenwandflächen können bei dieser Ausgestaltung zur Absorption der unerwünschten Strahlungsanteile ausgebildet werden.

Eine weitere Optimierung der spektralen Verteilung kann mit einem Absorptionsfilter (Farbglasfilter) erreicht werden, das der Reflexionsfilteranordnung in dem Nutzstrahlengang nachgeschaltet ist. Mit einem solchen Absorptionsfilter kann beispielsweise der gesamte sichtbare Anteil absorbiert werden, der sich noch in der Strahlung befindet. Die Verwendung eines Absorptionsfilters kann beispielsweise dann günstig sein, wenn befürchtet wird, dass das bestrahlte Hautmaterial durch den sichtbaren Strahlungsanteil zu stark erwärmt wird.

Mindestens ein Reflexionsfilter der Reflexionsfilteranordnung kann in seiner Winkellage relativ zu der Strahlungsquelle justierbar angeordnet sein. Es hat sich gezeigt, dass durch Veränderung der Winkellage eines Reflexionsfilters zur einfallenden Strahlung eine Wellenlängenverschiebung des Reflexionsbands bewirkt werden kann. Indem geeignete Justiermittel vorgesehen werden, die eine Einstellung eines oder mehrerer Reflexionsfilter der Reflexionsfilteranordnung in eine jeweils gewünschte Winkellage gestatten, ist eine bedarfsweise Abstimmung der Wellenlänge der von der erfindungsgemäßen Lampe letztendlich abgegebenen Nutzstrahlung möglich. Für die Justiermittel ist beispielsweise eine Schraubjustieranordnung vorstellbar.

Eine bevorzugte Ausführungsform der Erfindung sieht eine modulare Bauweise der Lampe vor, bei der die Lampe ein Lampengehäuse mit zwei lösbar miteinander verbindbaren Gehäuseteilen umfasst. Die Strahlungsquelle ist dabei an einem der Gehäuseteile angebracht, während die Reflexionsfilteranordnung und gewünschtenfalls auch ein der Reflexionsfilteranordnung im Nutzstrahlengang nachgeschaltetes Absorptionsfilter an dem anderen Gehäuseteil angebracht sind. Eine solche Bauweise der Lampe eröffnet die Möglichkeit, den mit der Reflexionsfilteranordnung bestückten Gehäuseteil als bedarfsweise austauschbares Modul auszuführen, wobei der Lampenhersteller eine Vielzahl solcher Module mit jeweils unterschiedlicher Reflexionscharakteristik bereitstellen kann. Der die Strahlungsquelle tragende Gehäuseteil kann dann nach Wunsch mit unterschiedlichen "Reflexionsmodulen" kombiniert werden, was der Lampe ein sehr vielfältiges Einsatzspektrum verschafft, ohne die Strahlungsquelle wechseln zu müssen. Die Formulierung eines Schutzbegehrens, das auf die Bereitstellung einer Vielzahl derartiger Reflexionsmodule mit baugleichem Gehäuseteil, jedoch unterschiedlicher Reflexionsfilteranordnung gerichtet ist, wird ausdrücklich vorbehalten. Sofern die Reflexionsfilteranordnung lösbar an ihrem zugehörigen Gehäuseteil angebracht ist, kann die Charakteristik der Lampe auch durch alleinigen Austausch eines oder mehrerer Reflexionsfilter der Reflexionsfilteranordnung variiert werden. In jedem Fall lässt sich durch Tausch eines oder mehrerer Reflexionsfilter - sei es im Rahmen des Austausches eines ganzen Reflexionsmoduls oder sei es im Rahmen des Austausches des betreffenden Filters bzw. der betreffenden Filter allein - ein beliebiger Nutzbereich aus dem breitbandigen Spektrum der Strahlungsquelle selektieren.

Die Erfindung wird nachfolgend anhand der beigefügten Zeichnungen weiter erläutert. Es stellen dar:
Fig. 1 eine Schnittansicht durch ein Ausführungsbeispiel einer erfindungsgemäßen Lampe,
Fig. 2 eine vergrößerte Schnittansicht einer als Strahlfalle dienenden Absorptionsfläche der Lampe der Fig. 1 und
Fig. 3 ein Diagramm, das beispielhaft einander überlappende Reflexionsbänder zweier Reflexionsspiegel einer erfindungsgemäßen Lampe zeigt.

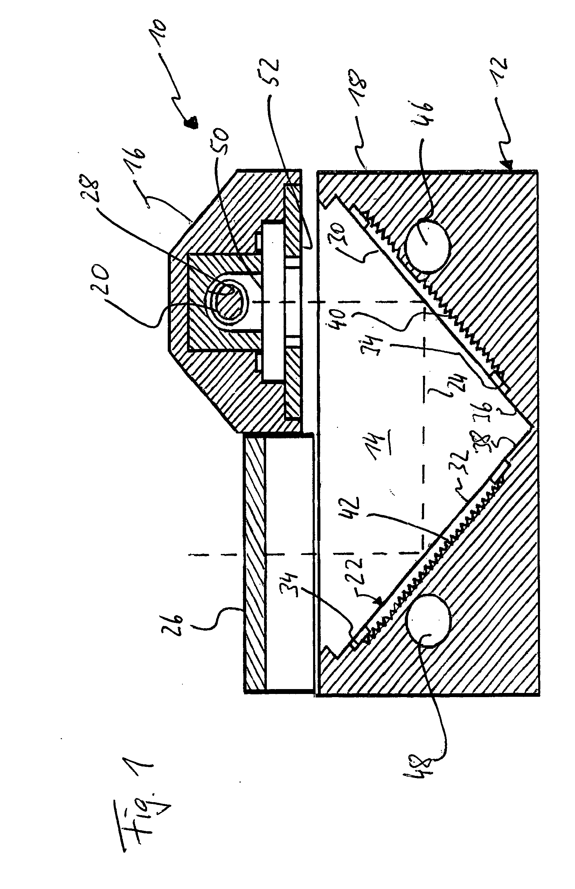

Die in Fig. 1 schematisch gezeigte Lampe ist allgemein mit 10 bezeichnet. Sie weist ein Lampengehäuse 12 auf, das einen Gehäusehohlraum 14 bildet. Das Gehäuse 12 weit zwei Gehäuseteile 16, 18 auf, deren einer (16) der Aufnahme und Halterung einer Strahlungsquelle 20 dient und deren anderer (18) eine Reflexionsfilteranordnung 22 enthält. Die Strahlungsquelle 20 ist eine breitbandige Strahlungsquelle, beispielsweise eine Xenon-Blitzleuchte, wobei beliebige andere breitbandige Strahlungsquellen genauso in Betracht kommen. Die Strahlungsquelle 20 ist in nicht näher dargestellter Weise elektrisch an eine Versorgungs- und Steuerschaltung angeschlossen bzw. anschließbar.

Die von der Strahlungsquelle 20 emittierte Strahlung wird entlang eines in Fig. 1 vereinfacht als Linie dargestellten Nutzstrahlengangs 24 zu einem Austrittsfenster 26 geleitet, an dem sie aus der Lampe 10 austritt. Entlang des Nutzstrahlengangs 24 wird die emittierte Strahlung durch die Reflexionsfilteranordnung 22 zweimal um jeweils annähernd 90° umgelenkt, sodass sie insgesamt um etwa 180° umgelenkt wird. Hinter der Strahlungsquelle 20 ist ein Reflektor 28, vorzugsweise ein Keramikreflektor, angeordnet, der für eine Erhöhung der Strahlungsausbeute sorgt.

Die Reflexionsfilteranordnung 22 umfasst im dargestellten Beispielfall zwei Multischicht-Reflexionsspiegel 30, 32, die beide eine hohe Reflektivität in einem vergleichsweise schmalbandigen Wellenlängenbereich aufweisen und außerhalb dieses Reflexionsbands mehr oder weniger stark transmissiv sind. Diese Eigenschaft der Reflexionsspiegel 30, 32 erlaubt es, aus dem emittierten Spektrum der Strahlungsquelle 20 ein interessierendes Nutzband zu extrahieren. Vorzugsweise ist die kombinierte Reflexionscharakteristik beider Reflexionsspiegel 30, 32 derart, dass sie UVB-Strahlung im Bereich zwischen 290 nm - 320 nm aus dem Emissionsspektrum der Strahlungsquelle 20 extrahieren. Dieser Wellenlängenbereich hat sich als günstig für die Behandlung von entzündlichen Hautkrankheiten wie Psoriasis und Vitiligo erwiesen. Es versteht sich, dass je nach Anwendungszweck der Lampe, also je nach Art der zu behandelnden Hautkrankheit oder der Art der kosmetischen Behandlung, andere Wellenlängenbereiche mittels geeignet abgestimmter Reflexionsspiegel selektiert werden können.

Die Reflexionsspiegel 30, 32 sind bei dem hier betrachteten Ausführungsbeispiel als 45°-Spiegel ausgebildet, die so in dem Gehäusehohlraum 14 angeordnet sind, dass die emittierte Strahlung der Strahlungsquelle 20 im wesentlichen unter diesem Winkel nacheinander auf die Spiegel 30, 32 trifft. Hochreflektierende Multischicht-Spiegel zeigen oftmals eine mehr oder weniger starke Empfindlichkeit der Mittenfrequenz ihres Reflexionsbands vom Einfallswinkel der Strahlung. Dies kann im Rahmen der Erfindung genutzt werden, um eine abstimmbare Lampe zu schaffen, deren abgestrahltes Nutzband veränderlich ist. Hierzu ist es vorstellbar, einen geeigneten Justiermechanismus in dem Lampengehäuse 12 vorzusehen, mittels dessen einer oder beide der Reflexionsspiegel 30, 32 in ihrer Winkellage justierbar sind.

Bei dem gezeigten Ausführungsbeispiel der Fig. 1 sind die Reflexionsspiegel 30, 32 jedoch winkelfest in das Gehäuse 12 eingebaut. Sie sind dabei in Ausnehmungen 34 eingesetzt, die in zwei annähernd unter einem Winkel von 90° zueinander stehenden Wandflächen 36, 38 des Gehäuseteils 18 ausgebildet sind. In diesen Ausnehmungen 34 sind die Reflexionsspiegel 30, 32 beispielsweise durch eine Schnapp-, Klebe- oder Schraubverbindung gehalten.

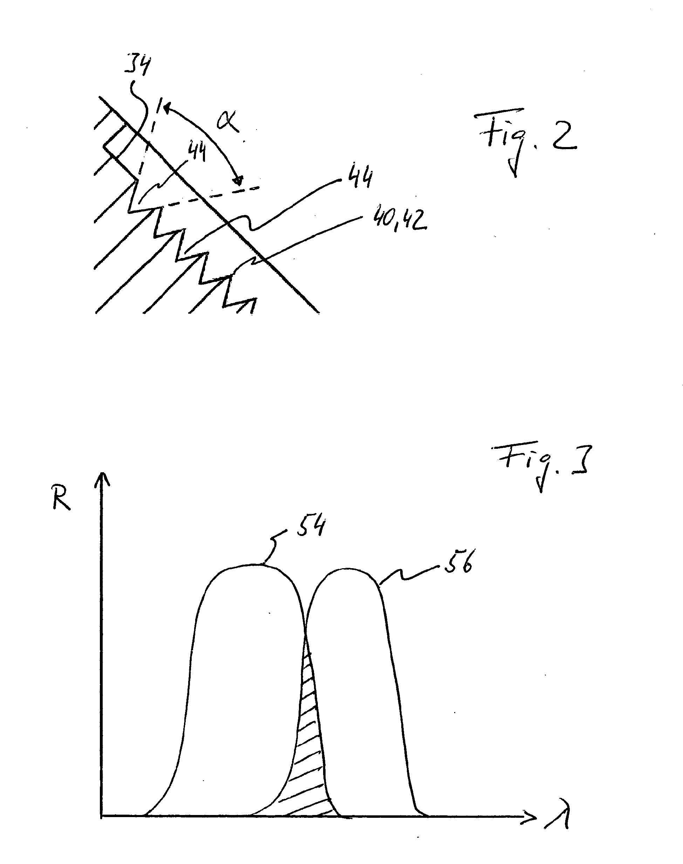

Die durch die Reflexionsspiegel 30, 32 hindurchgelassene, nicht genutzte Strahlung muss in dem Lampengehäuse 12 abgefangen und absorbiert werden. Dies geschieht im Beispielfall der Fig. 1 mit Hilfe profilierter Absorptionsflächen 40, 42, die am Grund der Ausnehmungen 34 hinter den Reflexionsspiegeln 30, 32 ausgebildet sind. Es wird nun zusätzlich auf Fig. 2 verwiesen. Die Absorptionsflächen 40, 42 sind mit einem Rillenprofil ausgeführt, nämlich in der Weise, dass sie eine Vielzahl parallel nebeneinander verlaufender Dreiecksrillen 44 aufweisen, deren Öffnungswinkel α vorzugsweise etwa 60° beträgt. Die Profilrillen 44 sind bevorzugt so in die Wandflächen 36, 38 bzw. die darin ausgebildeten Ausnehmungen 34 eingeformt, dass sie bei einer Querschnittsbetrachtung im wesentlichen symmetrisch zur einer gedachten Flächennormale der betreffenden Wandfläche 36 oder 38 ausgebildet sind. Die Rillenform der Profilierung in den Absorptionsflächen 40, 42 und der gewählte Öffnungswinkel der Rillen 44 führt dazu, dass sich die auf die Absorptionsflächen 40, 42 auftreffende Strahlung sozusagen in den Rillen 44 verliert und sie letztendlich im Material des Lampengehäuses 12 absorbiert wird und nicht in den Nutzstrahlengang 24 zurückgelangt. Statt der Rillenprofilierung der Absorptionsfläche 40, 42 kann selbstverständlich auch eine Profilierung dieser Flächen mit anderen Strukturen erfolgen, soweit hierdurch ein vergleichbarer Effekt erzielt wird.

Da über die Absorptionsflächen 40, 42 ein großer Teil der unerwünschten Wellenlängen absorbiert werden soll, empfiehlt sich für das Lampengehäuse 12, zumindest für den Gehäuseteil 18, ein Material mit hoher Temperaturbeständigkeit. Aluminium oder eine Aluminiumlegierung hat sich hierfür ais sehr gut geeignet erwiesen. Für eine noch bessere Absorption der unerwünschten Strahlung sind zumindest die Absorptionsflächen 40, 42, gewünschtenfalls die gesamten Innenwandflächen des Gehäuseteils 18, mit einem schwarzen temperaturbeständigen Lack überzogen. Beispielsweise kann hierfür ein Ofenlack auf Silikonharzbasis verwendet werden.

Um eine zu starke Erwärmung des Lampengehäuses 12 zu vermeiden, ist dieses von einer Kühlkanalanordnung durchsetzt, durch die ein Kühlmittel, beispielsweise deionisiertes Wasser, zirkulieren kann. Diesbezüglich sind in Fig. 1 zwei Kühlkanäle 46, 48 erkennbar, die in den Gehäuseteil 18 in geringem Abstand unterhalb der Absorptionsflächen 40, 42 eingearbeitet sind. Wenngleich nicht näher dargestellt, weist das Lampengehäuse 12 an seiner Außenseite geeignete Schnittstellenanschlüsse auf, die einen Anschluss der Lampe 10 an einen Kühlmittelkreislauf erlauben. Beispielsweise können solche Schnittstellenanschlüsse als einfache Steckanschlüsse ausgeführt sein, an die das Kühlmittel anliefernde und rücktransportierende Schläuche ansteckbar oder anschraubbar sind. Selbstverständlich kann das Lampengehäuse 12 von einem komplizierteren Geflecht von Kühlkanälen durchzogen sein, als es in Fig. 1 dargestellt ist. Die konkrete Ausgestaltung des Kühlkanalsystems wird sich nach der thermischen Belastung des Lampengehäuses 12 und der resultierenden erforderlichen Kühlleistung richten.

Das Austrittsfenster 26 ist als Farbglasfilter ausgebildet, welches etwaige verbleibenden unerwünschten Strahlungsanteile aus der von der Reflexionsfilteranordnung 22 reflektierten Strahlung entfernt. Da durch die Reflexionsfilteranordnung 22 bereits der größte Teil der unerwünschten Wellenlängen entfernt oder zumindest wesentlich geschwächt wird, ist der von dem Farbglasfilter 26 absorbierte Strahlungsanteil vergleichsweise gering. Absorptionsbedingte thermische Probleme sind deshalb bei dem Farbglasfilter 26 nicht zu befürchten.

Die emittierte Strahlung der Strahlungsquelle 20 wird mittels einer Quarzglasplatte 50 ausgekoppelt, die durch eine vorzugsweise justierbare Blende 52 an dem Gehäuseteil 16 gehalten ist. Die Quarzglasplatte 50 schützt die Strahlungsquelle 20 vor äußerer mechanischer Einwirkung sowie gegenüber dem Eindringen von Staub und Schmutz. Mittels der Blende 52 kann ein gewünschter Strahlungsquerschnitt eingestellt werden.

Der Gehäuseteil 16 kann mit der Strahlungsquelle 20, dem Reflektor 28, der Auskoppelplatte 50 und der Blende 52 eine vormontierte Baueinheit bilden, die auf den Gehäusetell 18 aufsetzbar und an diesem befestigbar ist. Der Gehäuseteil 18 besitzt im Beispielfall der Fig. 1 einen quaderförmigen Umriss, wobei die Wandflächen 36, 38 im wesentlichen unter einem Winkel von 45° zu den aufrecht stehenden äußeren Seitenflächen des Gehäuseteils 18 orientiert sind. Der Gehäuseteil 16 ist von oben auf den Gehäuseteil 18 aufgesetzt und erstreckt sich im wesentlichen nur über die Hälfte der Breite des Gehäuseteils 18. In der anderen Hälfte der Breite des Gehäuseteils 18 ist das Auftrittsfenster 26 angeordnet. Diese Ausgestaltung der Lampe 10 ermöglicht es, einen kurzen Abstand zwischen der Strahlungsquelle 20 und dem ersten Reflexionsspiegel 30 einzuhalten, gleichzeitig jedoch zu gewährleisten, dass die emittierte Strahlung der Strahlungsquelle 20 im wesentlichen ausschließlich zunächst auf den ersten Reflexionsspiegel 30 einfällt und nicht wesentliche Teile hiervon direkt auf den zweiten Reflexionsspiegel 32 gelangen. Die in Fig. 1 gezeigte Bauweise der Lampe 10 ermöglicht eine kompakte Ausführung derselben, insbesondere als Handlampe.

Soll die Lampe besonders schmalbandig ausgeführt werden, können die Reflexionsspiegel 30, 32 mittenversetzte, jedoch einander teilweise überlappende Reflexionsbänder besitzen. Diesbezüglich wird auf Fig. 3 verwiesen. Dort bezeichnet λ die Wellenlänge, während R das Reflexionsvermögen bezeichnet. Die Kurve 54 stellt schematisch ein Reflexionsband eines der Reflexionsspiegel 30, 32 dar, die Kurve 56 schematisch ein teilweise überlappendes Reflexionsband des anderen Reflexionsspiegels. Der gestrichelte Überlappungsbereich der beiden Reflexionsbänder stellt den Wellenlängenbereich der nach Reflexion an beiden Reflexionsspiegeln 30, 32 erhaltenen Strahlung dar. Durch einen derartigen Mittenversatz der Reflexionsbänder der Reflexionsspiegel 30, 32 kann ein außerordentlich schmales Nutzband aus dem Emissionsspektrum der Strahlungsquelle 20 selektiert werden.

Zur therapeutischen Behandlung der Haut wird die Lampe 10 vorzugsweise gepulst betrieben, wobei sich eine Pulsdauer der von der Strahlungsquelle 20 erzeugten Blitze im Bereich von 250 µs - 500 µs bei einer Pulsfrequenz zwischen 10 und 25 pro Sekunde als günstig erwiesen hat.

Durch Verwendung der Reflexionsspiegel 30, 32 lässt sich erreichen, dass die von der Lampe abgegebene selektierte Nutzstrahlung nur unwesentlich abgeschwächt ist. Es hat sich gezeigt, dass die durch die Reflexionsspiegel 30, 32 und das Absorptionsfilter 26 bewirkte Intensitätsabwächung der Nutzstrahlung beispielsweise bei lediglich etwa 10% liegen kann. Gleichzeitig bewirkt die Hintereinanderschaltung der Reflexionsfilter 30, 32 und des Absorptionsfilters 26 eine nahezu vollständige Beseitigung aller unerwünschten Wellenlängen, sodass ein sehr hohes Kontrastverhältnis der Nutzstrahlungsintensität zur Störstrahlungsintensität, d.h. der Intensität der unerwünschten Strahlungsanteile, erzielt wird. Je nach Wahl der Reflexionsfilter 30, 32 und des Absorptionsfilters 26 kann eine Nutzbandbreite von nur einigen wenigen Nanometern, beispielsweise 5 oder 10 Nanometern erzielt werden, wobei das Nutzspektrum ausgesprochen scharf begrenzt sein kann. Es ist freilich genauso möglich, durch entsprechende Wahl der verschiedenen Filter ein breiteres Nutzspektrum aus dem von der Strahlungsquelle bereitgestellten Spektrum zu selektieren, beispielsweise einen Wellenlängenbereich mit einer Breite von 50 oder 100 nm.

## Patentansprüche

1. Lampe zur Behandlung der Haut, umfassend
- eine Breitband-Strahlungsquelle (20),
- eine Reflexionsfilteranordnung (22), welche einen Teil der von der Strahlungsquelle (20) erzeugten Strahlung entlang eines Nutzstrahlengangs (24) reflektiert, sowie
- eine Strahlfalle (40, 42), welche die durch die Reflexionsfilteranordnung (22) durchgehende Strahlung absorbiert.

2. Lampe nach Anspruch 1,
**dadurch gekennzeichnet, dass** die Strahlfalle mindestens eine profilierte Absorptionsfläche (40, 42) umfasst.

3. Lampe nach Anspruch 2,
**dadurch gekennzeichnet, dass** in die profilierte Absorptionsfläche (40, 42) eine Vielzahl von parallel nebeneinander verlaufenden Rillen (44) eingeformt ist.

4. Lampe nach Anspruch 3,
**dadurch gekennzeichnet, dass** die Rillen (44) im Querschnitt als Dreiecksrillen ausgebildet sind.

5. Lampe nach Anspruch 4,
**dadurch gekennzeichnet, dass** der Öffnungswinkel der Dreiecksrillen (44) zwischen 45 und 75 Grad, vorzugsweise etwa 60 Grad beträgt.

6. Lampe nach einem der Ansprüche 2 bis 5,
**dadurch gekennzeichnet, dass** die Absorptionsfläche (40, 42) an der Innenwand (36, 38) eines Lampengehäuses (12), insbesondere aus Aluminium, ausgebildet ist, welches von einer Kühlkanalanordnung (46, 48) durchsetzt ist und Kühlmittelschnittstellenanschlüsse für den Anschluss an einen Kühlmittelkreislauf aufweist.

7. Lampe nach einem der vorhergehenden Ansprüche,
**dadurch gekennzeichnet, dass** die Reflexionsfilteranordnung (22) mindestens und vorzugsweise insgesamt zwei im Nutzstrahlengang (24) hintereinander angeordnete Reflexionsfilter (30, 32) aufweist.

8. Lampe nach Anspruch 7,
**dadurch gekennzeichnet, dass** die zwei Reflexionsfilter (30, 32) an zwei schräg zueinander verlaufenden Innenwandflächen (36, 38) eines Lampengehäuses (12) der Lampe angeordnet sind, insbesondere in Ausnehmungen (34) in den Innenwandflächen gehalten sind.

9. Lampe nach Anspruch 7 oder 8,
**dadurch gekennzeichnet, dass** die zwei Reflexionsfilter (30, 32) mittenversetzte, einander teilweise überlappende Reflexionsbänder aufweisen.

10. Lampe nach einem der vorhergehenden Ansprüche,
ferner **gekennzeichnet durch** ein der Reflexionsfilteranordnung (22) nachgeschaltetes Absorptionsfilter (26) in dem Nutzstrahlengang.

11. Lampe nach einem der vorhergehenden Ansprüche,
**dadurch gekennzeichnet, dass** die Strahlungsquelle (20) von einer Xenon-Blitzleuchte gebildet ist.

12. Lampe nach einem der vorhergehenden Ansprüche,
**dadurch gekennzeichnet, dass** mindestens ein Reflexionsfilter der Reflexionsfilteranordnung (22) in seiner Winkellage relativ zu der Strahlungsquelle (20) justierbar angeordnet ist.

13. Lampe nach einem der vorhergehenden Ansprüche,
**dadurch gekennzeichnet, dass** die Lampe ein Lampengehäuse (12) mit zwei lösbar miteinander verbindbaren Gehäuseteilen (16, 18) umfasst, an deren einem (16) die Strahlungsquelle (20) angebracht ist und an deren anderem (18) die Reflexionsfilteranordnung (22), gewünschtenfalls auch ein der Reflexionsfilteranordnung nachgeschaltetes Absorptionsfilter (26), angebracht ist.
